# EUROPEAN PATENT SPECIFICATION

(11) **EP 0 640 092 B1**
(45) Date of publication and mention of the grant of the patent: **11.12.1996**
(21) Application number: 93909461.1
(22) Date of filing: 01.04.1993
(51) Int. Cl.: C07H 19/06

(54) **A PROCESS FOR THE PREPARATION OF RIBONUCLEOTIDE REDUCTASE INHIBITORS**
VERFAHREN ZUR HERSTELLUNG VON RIBONUKLEOTID-REDUKTASEHEMMERN
PROCEDE DE PREPARATION D'INHIBITEURS DE RIBONUCLEOTIDE-REDUCTASE

(30) Priority: 12.05.1992 US 881978; 26.03.1993 US 31012
(43) Date of publication of application: 01.03.1995
(73) Proprietor: MERRELL PHARMACEUTICALS INC., Cincinnati Ohio 45215 (US)
(72) Inventor: McCARTHY, James, R., West Chester, OH 45069 (US); MATTHEWS, Donald, P., West Chester, OH 45069 (US); SABOL, Jeffrey, S., Loveland, OH 45140 (US)
(74) Representative: VOSSIUS & PARTNER
(86) International application number: US9303023
(87) International publication number: WO9323414

(56) References cited:
- EP-A- 0 365 849
- EP-A- 0 372 268
- TETRAHEDRON LETTERS. vol. 31, no. 38, 1990, OXFORD GB pages 5449 - 5452 MCCARTHY J.R. ET AL 'A new route to vinyl sulfones'
- JOURNAL OF THE AMERICAN CHEMICAL SOCIETY. vol. 113, 1991, GASTON, PA US pages 7439 - 7440 MCCARTHY J.R. ET AL 'Stereospecific Method to E and Z Terminal
- Fluoro Olefins and Its Application to the Synthesis of 2'-Deoxy-2'- fluoromethylene Nucleosides as Potential Inhibitors of Ribonucleoside Diphosphate Reductase' See scheme 1
- TETRAHEDRON LETTERS. vol. 33, no. 22, 26 May 1992, OXFORD GB pages 3101 - 3104 SABOL J.S. AND MCCARTHY J.R. 'A new route to 1,1-difluoro olefins: Application to the synthesis of 2'-deoxy-2'difluoromethylene nucleosides'

## Description

The present invention relates to a novel process for the preparation of ribonucleotide reductase inhibitors. The target compounds of the novel process described in this application are disclosed by McCarthy et al. in European Patent Application Publication No. 0 372 268 published June 13, 1990 which related to certain novel 2'-halomethylidene, 2'-ethenylidene and 2'-ethynyl cytidine, uridine and guanosine derivatives and compositions thereof, which are useful in the treatment of patients afflicted with neoplastic or viral disease states. The previous synthesis of these compounds involved a seven step reaction sequence in which a uridine derivative was the required starting material. The uridine derivative was converted to the known ketone intermediate of structure (A) 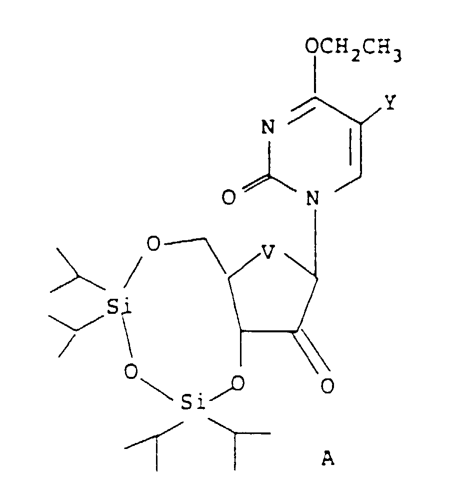 wherein V is oxy or methylene and Y is hydrogen, C₁-C₄ alkyl or C₁-C₄ alkoxy, following generally the procedure of Matsuda et al. [Chem. Pharm. Bull. 1988, 36(3), 945] in a four step process that was labor intensive and involved multiple chromatographies. This intermediate was then subjected to olefination at the 2'-carbonyl to produce the exocyclic fluorovinyl sulfone followed by stannylation to provide the exocylic (fluorovinyl) stannane. In the final step this compound was destannylated and the 3',5'-hydroxyls were deprotected to provide the desired ribonucleotide reductase inhibitor in low yield.

The novel process of the present invention utilizes a cytidine derivative as the required starting material and provides the ribonucleotide reductase inhibitor in a more efficient five step reaction sequence. The process is more efficient than the process of McCarthy et al. in that it requires fewer chromatographies and results in an overall yield of greater than 25% for the five steps.

The present invention provides a novel process for preparing a compound of the formula 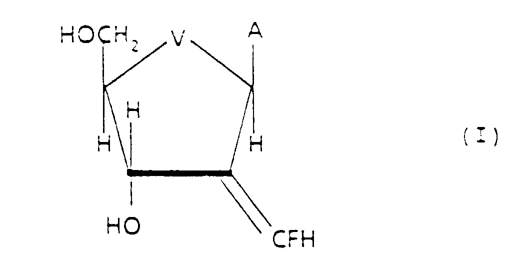 wherein
- V: is oxy or methylene and
- A: is a radical of the formula 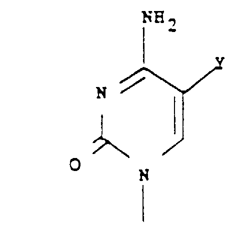
wherein Y is hydrogen, C₁-C₄ alkyl or C₁-C₄ alkoxy comprising the steps of:
(a) reacting a compound of the formula 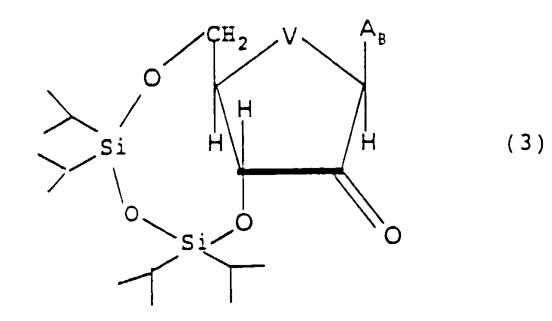 wherein A_{B} is a radical of the formula 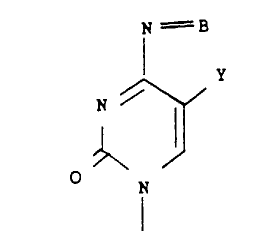 and B is a suitable nitrogen blocking group of the formula CR₁NR₂R₃ wherein R₁ is a hydrogen or C₁-C₄ alkyl and R₂ and R₃ are independently C₁-C₄ alkyl, with a suitable phosphonate ylid of the formula (X)₂OP=CF(SO₂Ar) wherein Ar is an aryl group and X is a phenoxy or C₁-C₄ alkoxy to produce an exocyclic fluorovinyl sulfone;
(b) reacting the exocyclic fluorovinyl sulfone with a suitable base or suitable weak acid to produce a deprotected amine;
(c) reacting the deprotected amine with a stannylating reagent of the formula (R)₃SnH wherein R is aryl or C₁-C₄ alkyl to produce an exocyclic (fluorovinyl)stannane;
(d) reacting the exocyclic (fluorovinyl)stannane with a protolysis agent and, either concomitantly or sequentially, reacting the silyl protecting group with a suitable acid or a fluoride ion source.

The present invention further provides a novel process for preparing a compound of the formula 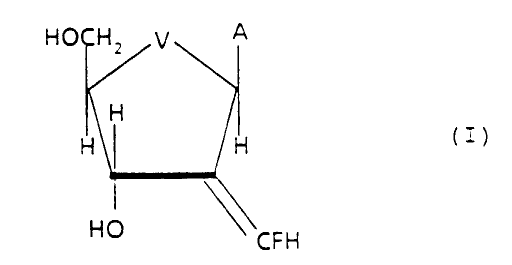 wherein
- V: is oxy or methylene and
- A: is a radical of the formula 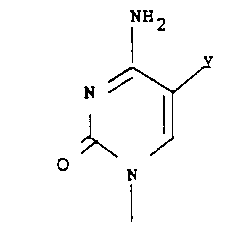
wherein Y is hydrogen, C₁-C₄ alkyl or C₁-C₄ alkoxy comprising the steps of:
(a) reacting a compound of the formula 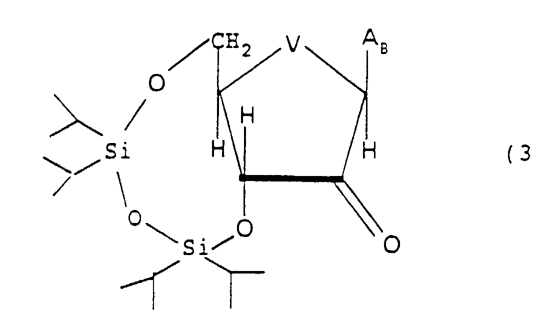 wherein A_{B} is a radical of the formula 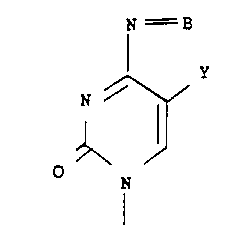 and B is a suitable nitrogen blocking group of the formula CR₁NR₂R₃ wherein R₁ is a hydrogen or C₁-C₄ alkyl and R₂ and R₃ are independently C₁-C₄ alkyl, with a suitable base or suitable weak acid to produce a deprotected amine;
(b) reacting the deprotected amine with a suitable phosphonate ylid of the formula (X)₂OP=CF(SO₂Ar) wherein Ar is an aryl group and X is a phenoxy or C₁-C₄ alkoxy to produce an exocyclic fluorovinyl sulfone;
(c) reacting the exocyclic fluorovinyl sulfone with a stannylating reagent of the formula (R)₃SnH wherein R is aryl or C₁-C₄ alkyl to produce an exocyclic (fluorovinyl)stannane;
(d) reacting the exocyclic (fluorovinyl)stannane with a protolysis agent and, either concomitantly or sequentially, reacting the silyl protecting group with a suitable acid or a fluoride ion source.

In a further embodiment the present invention provides novel compounds of the following formulas

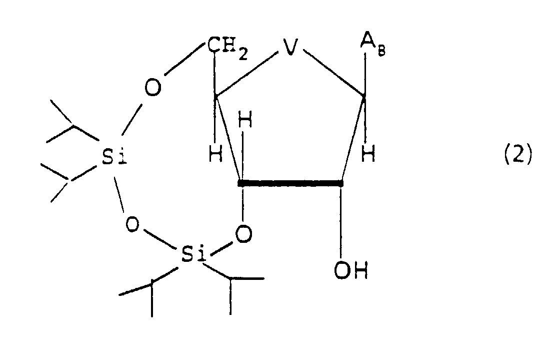

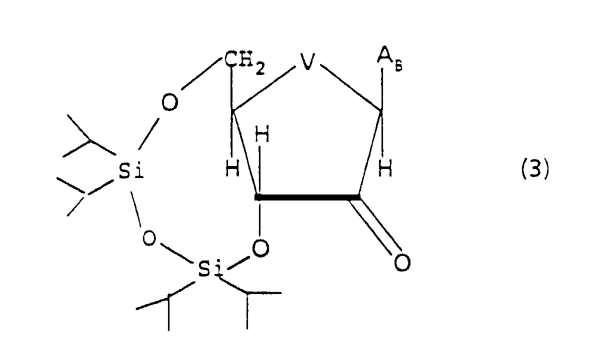

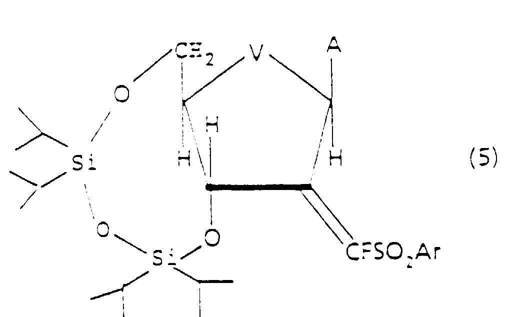

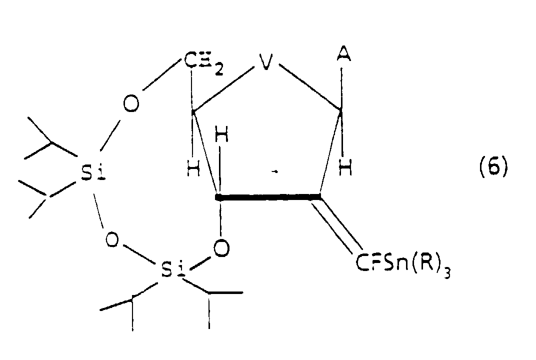

As used herein the term "C₁-C₄ alkyl" refers to a saturated straight or branched chain hydrocarbon radical of one to four carbon atoms. Included within the scope of this term are methyl, ethyl, n-propyl, isopropyl, n-butyl, isobutyl and the like. The term "C₁-C₄ alkoxy" refers to an alkyloxy radical made up of an oxygen radical bearing a saturated straight or branched chain hydrocarbyl radical of one to four carbon atoms and specifically includes methoxy, ethoxy, propyloxy, isopropyloxy, n-butyloxy, isobutyloxy, sec-butyloxy, tertiary butyloxy and the like. The term "halogen" or "halo" refers to a fluorine, chlorine, bromine, or iodine atom. The term "Ar" or "aryl" refers to an aromatic radical of from about 6 to 12 carbon atoms, such as phenyl, naphthyl or phenyl(C₁-C₄)alkyl groups, wherein said groups are optionally substituted with one, two or three substituents selected from the group consisting of C₁-C₄ alkyl, halo-substituted C₁-C₄ alkyl, halogen or C₁-C₄ alkoxy. The term "phenyl(C₁-C₄)alkyl" refers to a phenyl group substituted with a C₁-C₄ alkyl including phenylmethyl, phenethyl and the like.
Specifically included within the scope of the term "Ar" or "aryl" are phenyl, p-toluyl, p-methoxyphenyl, p-chlorophenyl, naphthyl and the like.

The general synthetic process of the present invention is set forth in Scheme A. All the substituents, unless otherwise indicated, are previously defined. The reagents and starting materials for use in this process are readily available to one of ordinary skill in the art.

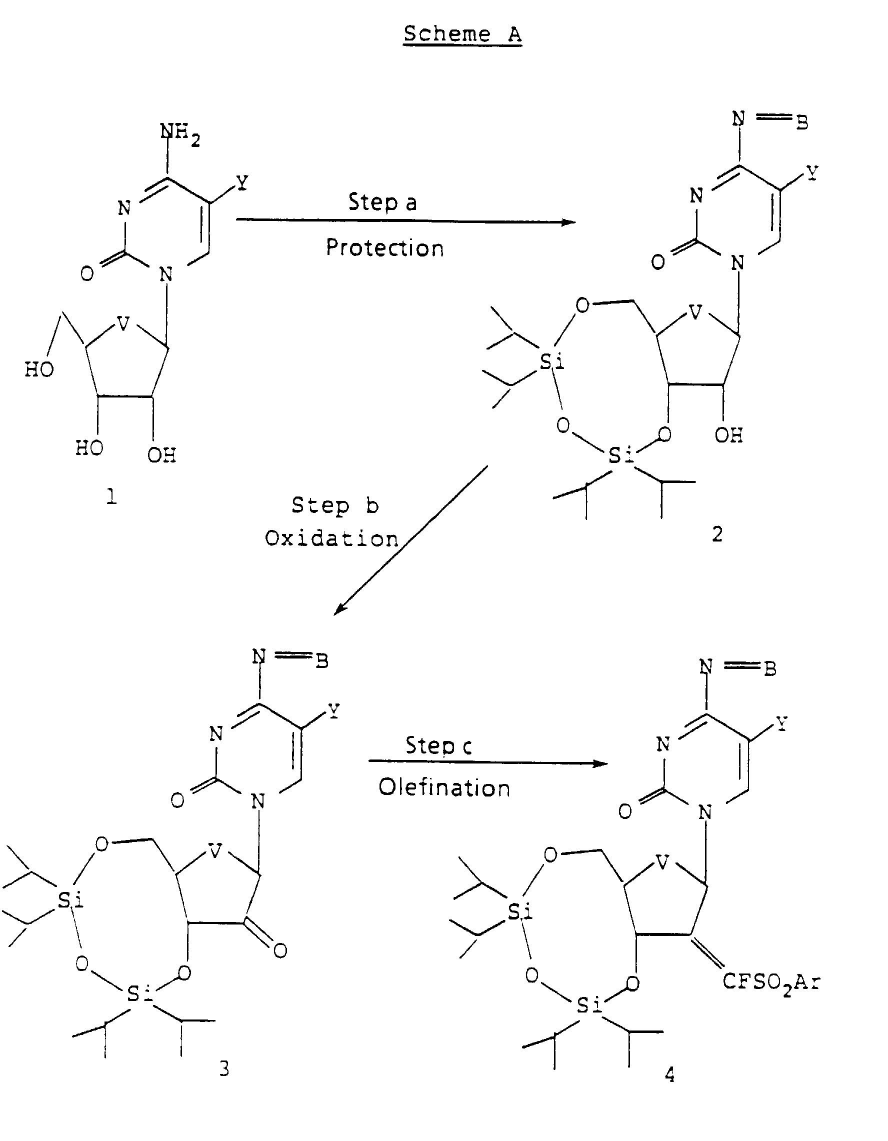

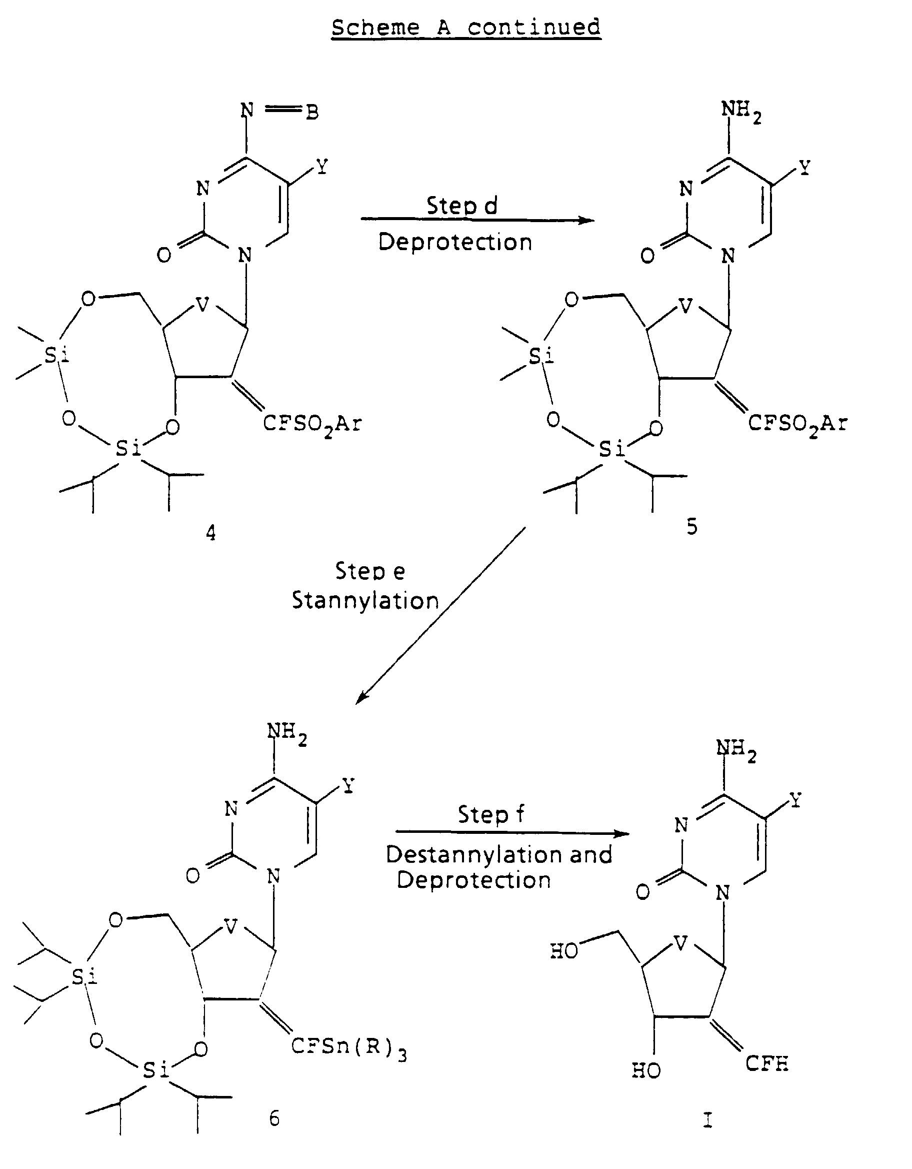

In Scheme A, step a, the 3' and 5' hydroxyls of the appropriately substituted cytidine derivative of structure (1) are protected as the 1,3-(1,1,3,3-tetraisopropyldisiloxanylidene) derivative and the amino function is protected with a suitable nitrogen blocking group to provide the compound defined by structure (2). A suitable nitrogen blocking group is bound to nitrogen as the imine derivative and is stable to oxidizing conditions such as methyl sulfoxide/oxalyl chloride and to strong organic bases, such as lithium diisopropylamide. For example, suitable nitrogen blocking groups would be N-(N',N'-dimethylaminomethylene)amine, N-(methyl-N',N'-dimethylaminomethylene)amine, N-(methyl-N',N'-diethylaminomethylene)amine, N-(ethyl-N',N'-diethylaminomethylene)amine, and the like. The preferred nitrogen blocking is N-(N',N'-dimethylaminomethylene)amine.

More specifically, in Scheme A, step a, the cytidine derivative (1) is treated with an equivalent of 1,3-dichloro-1,1,3,3-tetraisopropyldisiloxane in a basic organic solvent, such as pyridine and allowed to stir for 12 to 24 hours at approximately 10°C to 30°C. An excess of dimethylformamide dimethyl acetal is then added to the reaction which is allowed to stir for 2 to 6 hours. The solvent is removed under vacuum and the residue is purified by techniques well known to one skilled in the art to provide the compound defined by structure (2).

In Scheme A, step b, the 2'-hydroxyl group is oxidized to the ketone derivative defined by structure (3) by oxidation methods well known to one skilled in the art.

For example, approximately 1.5 equivalents of oxalyl chloride is dissolved in a suitable anhydrous organic solvent, such as methylene chloride, and cooled to about -75°C. To this solution is added 3 equivalents of methyl sulfoxide dropwise, maintaining the temperature below -55°C. An equivalent of the product defined by structure (2) is dissolved in a suitable amount of anhydrous organic solvent, such as methylene chloride, and added slowly to the reaction with stirring. After addition is complete the reaction is stirred for approximately 30 minutes at -75°C, an excess of a suitable organic base, such as triethylamine, is added and the reaction is allowed to warm to room temperature. The ketone derivative (3) is then isolated and purified by techniques well known to one skilled in the art. For example, silica gel chromatography followed by recrystallization from a suitable organic solvent or solvent mixture, such as 10% chloroform/hexane provides the ketone derivative (3).

In Scheme A, step c, the ketone derivative (3) can be olefinated to yield the corresponding exocyclic fluorovinyl sulfone (4) by reaction with a phosphorus ylide which can be prepared according to procedures which are well known and appreciated in the art of chemistry as described by March ["Advanced Organic Chemistry: Reactions, Mechanisms and Structure", McGraw-Hill Book Company, 2nd Ed., 1977, 864-872].

More specifically, olefination may be performed by reacting the appropriately substituted ketone derivative (3) with a suitable phosphonate ylide of formula (X)₂OP=CF(SO₂Ar) through a modification of the Wittig reaction as described by Wadsworth et al. [J. Am. Chem. Soc. 1961, 83,1733]. For example, the appropriately substituted phosphonate of formula (X)₂OPCHF(SO₂Ar) is dissolved in a suitable anhydrous organic solvent and cooled to approximately -70°C. A suitable anhydrous organic solvent includes hydrocarbon solvents, dialkyl ethers, C₁-C₆ alkanes, tetrahydrofuran and the like. The preferred anhydrous organic solvent is tetrahydrofuran. An equivalent of a strong base is added slowly to produce the ylide. A wide variety of bases can be used including alkoxides and organometallics, such as alkyllithium, lithium dialkylamide, sodium hydride and the like. The preferred bases are potassium tert-butoxide and lithium bis(trimethylsilyl)amide. After approximately one hour an equivalent of the appropriately substituted ketone derivative (3) is added to the phosphonate ylide at approximately -60°C followed by warming to about 0°C for about 30 minutes and then warmed to room temperature for approximately 2.5 hours. The exocyclic fluorovinyl sulfone (4), is then isolated and purified by techniques well known to one skilled in the art. For example, the reaction is quenched with saturated ammonium chloride and the aqueous is extracted with a suitable organic solvent, such as diethyl ether. The organic phase is dried over a suitable drying agent, such as anhydrous magnesium sulfate, filtered and concentrated under vacuum. The crude material is filtered through silica gel with a suitable organic solvent, such as ethyl acetate to provide the exocyclic fluorovinyl sulfone (4).

In Scheme A, step d, deprotection of the exocyclic fluorovinyl sulfone (4) by removal of the nitrogen blocking group to provide the appropriately substituted deprotected amine derivative (5) is performed by dissolving the protected compound in an organic solvent, such as dioxane followed by treatment with an excess of a suitable base. A suitable base is one that is capable of removing the nitrogen blocking group without removing the silyl protecting group at the 3', 5' positions. Examples of suitable bases are ammonium hydroxide, ammonia, methylamine and the like. The preferred suitable base is ammonium hydroxide. The reaction is stirred for about 8 to 24 hours at room temperature and the deprotected amine (5) is isolated and purified by techniques well known to one skilled in the art. For example, the solvent is removed under vacuum azeotroping off the water with addition of ethanol and the crude material is purified by flash chromatography using a suitable solvent mixture, such as 5% hexane/ethyl acetate to provide the deprotected amine (5).

An alternative procedure for preparation of the deprotected amine (5) is performed by dissolving the exocyclic fluorovinyl sulfone (4) in an organic solvent, such as ethyl acetate and treating with one equivalent of concentrated ammonium hydroxide for approximately 2 hours at room temperature. The deprotected amine (5) is isolated and purified by techniques well known to one skilled in the art. For example, the solvent is removed under vacuum azeotroping off the water with addition of ethanol and the crude material is purified by flash chromatography using a suitable solvent mixture, such as 5% hexane/ethyl acetate to provide the deprotected amine (5).

In Scheme A, step e, stannylation of the deprotected amine (5) utilizing procedures which are known to one of ordinary skill in the art as described by McCarthy et al. [J. Am. Chem. Soc., 1991, 113, 7439] provides the exocyclic (fluorovinyl)stannane of structure (6). For example, the deprotected amine (5) is dissolved in a suitable organic solvent, such as benzene and treated with an excess of a suitable stannylating reagent of formula (R)₃SnH. Suitable stannylating reagents are tributyltin hydride, triethyltin hydride, trimethyltin hydride, triphenyltin hydride and the like. The preferred stannylating reagent is tributyltin hydride. The reaction is then initiated by employing a suitable initiator. Suitable initiators are azobisisobutyronitrile (AIBN), UV light, heat and the like. The preferred suitable initiator is azobisisobutyronitrile (AIBN). A catalytic amount of AIBN is added and the reaction is refluxed for approximately 18 hours. The product is then isolated and purified by techniques well known to one skilled in the art to provide the exocyclic (fluorovinyl)stannane defined by structure (6). For example, the reaction is concentrated under vacuum and the residue is purified by flash chromatography using a suitable solvent mixture, such as 4% to 6% methanol/methylene chloride to provide exocyclic (fluorovinyl)stannane (6).

In Scheme A, step f, the exocyclic (fluorovinyl)stannane (6) can be sequentially converted to the exocyclic fluorovinyl derivative of Formula I by first destannylating with a protolysis agent under mild conditions in the absence of a fluoride ion source. A suitable protolysis agent will substitute a proton for the stannane substituent. Examples of a protolysis agent are ammonia/methanol, silica gel and the like. The protected exocyclic vinylfluoride is then deprotected by treatment with a suitable acid, such as aqueous hydrochloric acid or a fluoride ion source to provide the exocyclic vinylfluoride of Formula I. Examples of a fluoride ion source are sodium fluoride, potassium fluoride, cesium fluoride, tetrabutylammonium fluoride, ammonium fluoride and the like. The preferred fluoride ion source is potassium fluoride.

For example, in Scheme A, step f, the exocyclic (fluorvinyl)stannane (6) is combined with excess silica gel as described by Stork et al. [ J. Arm. Chem. Soc. 1987, 109, 2829] in a suitable organic solvent, such as methanol and allowed to stir until removal of the tributyltin is complete. The protected exocyclic vinylfluoride is then isolated and purified by techniques well known to one skilled in the art, such as flash chromatography. This product is then treated with an excess of a fluoride ion source, such as tetrabutylammonium fluoride, in a suitable organic solvent, such as methanol and allowed to stir until the deprotection is complete. The product is then isolated and purified by techniques well known to one of ordinary skill in the art to provide the exocyclic fluorovinyl compound defined by Formula I. For example, the reaction is concentrated under vacuum and purified by flash chromatography using a suitable solvent mixture, such as 50% ethyl acetate/hexane followed by 10% to 20% methanol/ethyl acetate. Recrystallization from methanol/ethyl acetate provides the compound of Formula I.

In Scheme A, step f, the exocyclic (fluorovinyl)stannane (6) can also be destannylated and deprotected concomitantly by reacting it with a protolysis agent and fluoride ion source or a suitable acid to provide the exocyclic vinylfluoride of Formula I.

For example, in Scheme A, step f, the exocyclic (fluorovinyl)stannane (6) is dissolved in a suitable organic solvent, such as methanol, treated with a protolysis agent such as potassium fluoride, which also acts as a fluoride ion source and the reaction is heated to reflux for approximately 24 hours. After cooling, the solvent is partially concentrated and excess silica gel is added. The remaining solvent is removed and the product is isolated and purified by techniques well known to one of ordinary skill in the art to provide the compound defined by Formula I. For example, the reaction is concentrated under vacuum and purified by flash chromatography using a suitable solvent mixture, such as 50% ethyl acetate/hexane followed by 10% to 20% methanol/ethyl acetate.
Recrystallization from methanol/ethyl acetate provides the compound of Formula I.

Of course one skilled in the art would understand that the stepwise synthesis as presented in Scheme A is not limited to the particular sequence of steps as presented.

For example, in Scheme A, step c the olefination reaction may be performed subsequent to the deprotection reaction performed in step d.

The appropriately substituted phosphonate of formula (X)₂OPCHF(SO₂Ar) required for preparation of the phosphonate ylide for reaction in Scheme A, step c can be obtained by a variety of methods described by Schemes B, C and D.

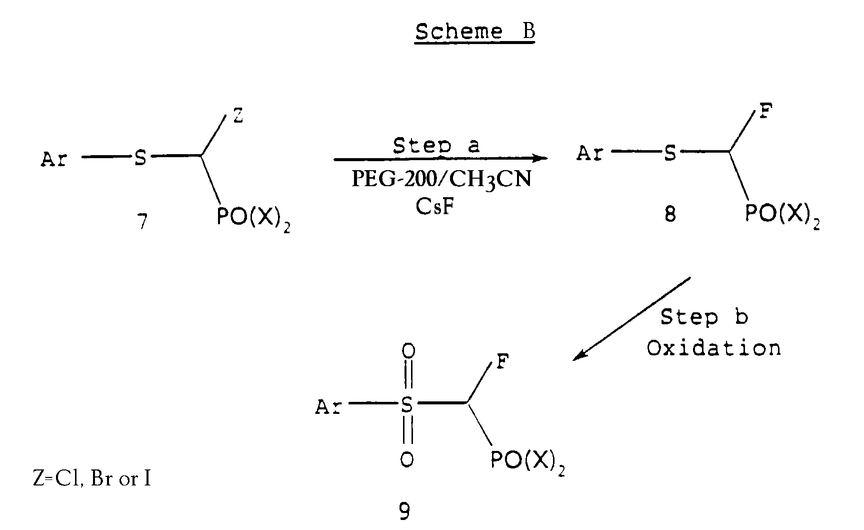

For example in Scheme B, step a, the compound defined by structure (7) is dissolved in a mixture of poly(ethylene glycol):acetonitrile in a ratio of approximately 1:2. A suitable molecular weight range for the poly(ethylene glycol) is between 100 and 400 g/mol. An excess of a fluoride ion source, such as cesium fluoride is added and the reaction is heated to approximately 80°C for 1 to 24 hours. The reaction is then diluted with water and the product extracted with a suitable organic solvent such as chloroform to provide after drying and concentrating under vacuum the product defined by structure (8). This is then oxidized by techniques well known to one skilled in the art. For example, treatment of compound (8) with potassium peroxymonosulfate in a suitable organic solvent, such as methanol provides the appropriately substituted phosphonate defined by structure (9).

The appropriately substituted phosphonate defined by structure (9) can be obtained by another method as described in Scheme C.

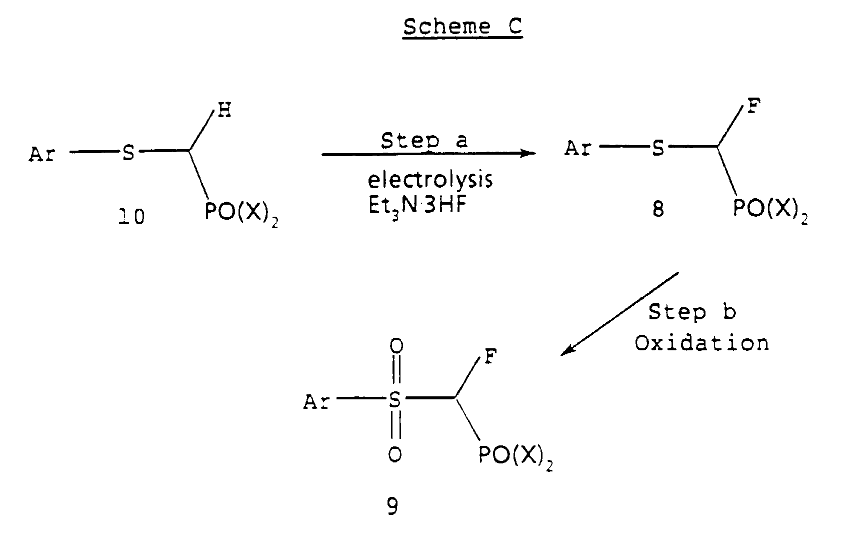

In Scheme C, step a, the compound defined by structure (10) is dissolved in a suitable organic solvent, such as tetrahydrofuran, treated with excess triethylamine trihydrofluoride and the solution is cooled to approximately -78°C. The solution is then subjected to a controlled potential electrolysis for about 3 to 10 hours to effectuate anodic monofluorination following generally the procedure of Matsue et al. [ J. Chem. Soc., Chem. Commun. 1991, 1028]. The product defined by structure (8) is then isolated and oxidized in step b as described in Scheme B to provide the appropriately substituted phosphonate defined by structure (9).

Additionally, the appropriately substituted phosphonate defined by structure (9) may be prepared in situ as the ylide defined by the formula (X)₂OP=CF(SO₂Ar) as shown in Scheme D.

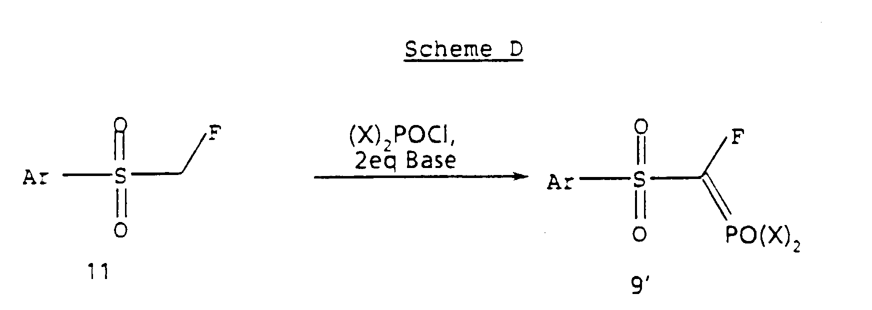

In Scheme D, the appropriately substituted sulfone, such as fluoromethylphenyl sulfone, which can be prepared according to McCarthy et al. [Tetrahedron Lett. 1990, 31, 5449], is dissolved in a suitable anhydrous organic solvent, such as tetrahydrofuran, cooled to approximately -70°C and treated with an equivalent of a dialkyl chlorophosphate, such as diethyl chlorophosphate, defined by the formula (X)₂POCl. The solution is then treated slowly with 2 equivalents of a strong base, such as lithium bis(trimethylsilyl)amide. After addition is complete the reaction is stirred at approximately -65°C for about 1 hour to provide the ylide defined by structure (9').

The following examples present typical syntheses as described by Schemes A, B, C and D. These examples are understood to be illustrative only and are not intended to limit the scope of the invention in any way. As used in the following examples, the following terms have the meanings indicated: "g" refers to grams, "mmol" refers to millimoles, "mL" refers to milliliters, "°C" refers to degrees Celsius, "TLC" refers to thin layer chromatographic, "mg" refers to milligrams, "µL" refers to microliters and "δ" refers to parts per million downfield from tetramethlysilane.

### Example 1

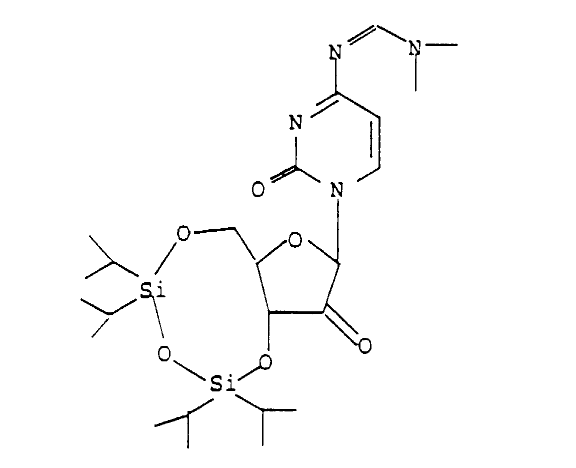

### 2'-deoxy-N-[(dimethylamino)amino)methylene]-2'-oxo-3',5'-O-[1,1,3,3-tetrakis(1-methylethyl)-1,3-disiloxanediyl] cytidine.

Scheme A, step a; In a one-neck 2 L round bottom flask equipped with an addition funnel and under a nitrogen atmosphere, treat a slurry of cytidine (100 g, 0.41 mol) in anhydrous pyridine (800 mL) with 1,3-dichloro-1,1,3,3-tetraisopropyldisiloxane (130 g, 0.41 mol). After 30 minutes, everything dissolves. Stir the reaction overnight at room temperature. Add neat dimethylformamide dimethyl acetal (165 g, 1.38 mol) and stir the reaction for 4 hours. The reaction will warm slightly and become cloudy. Remove the pyridine under high vacuum and azeotrope with toluene (2 X 500 mL) to remove any remaining pyridine. Heat the solid residue with 1 L of ethyl acetate and gravity filter into a 5 L flask. Dissolve the insolubles in water (800mL) and brine (200mL) and extract additional product into ethyl acetate (2 X 600 mL). Dry the solution over anhydrous magnesium sulfate and concentrate under vacuum to provide 90g of "wet" solid. Dissolve this solid in ethyl acetate (200mL) and combine with the previous ethyl acetate filtrate. Add hexane (3 L) to the solution, heat and filter while still hot. Allow the solution to sit overnight. Collect the white crystals which form by filtration and dry in a warm vacuum oven to produce compound (142.4 g, 64%). Concentrate the above filtrate and purify the residue by flash chromatography on 1.4 L silica gel (12.5% ethanol/ethyl acetate) to yield an additional amount of compound (17.4 g) from fractions 8-16. Fractions 2-7 contain compound contaminated with pyridine. Concentrate these fractions under vacuum and recrystallize the residue to provide an additional amount of compound (24.5 g) to provide a total amount of 184.2 g (83% yield) of N-[(dimethylamino)methylene]-3',5'-O-[1,1,3,3-tetrakis(1-methylethyl)-1,3-disiloxanediyl]-cytidine as white crystals, mp 137-138°C;
¹H NMR (CDCl₃) δ 0.97-1.10 (m, 28), 3.14 (s, 3), 3.16 (s, 3), 3.98-4.39 (m, 5), 5.82 (s, 1), 6.05 (d, 1, J = 7.2 Hz), 7.92 (d, 1, J= 7.5 Hz), 8.85 (s, 1); MS (CI/CH₄) m/z 541 (MH⁺).
Anal. Calcd for C₂₄H₄₄N₄O₆Si₂: C, 53.30; H, 8.20; N, 10.36.
Found: C, 52.93; H, 8.33; N, 10.07.

Scheme A, step b; Flush a 3 neck 2 L flask fitted with a condenser, mechanical stirrer and a thermometer with nitrogen and charge with oxalyl chloride (13.08 mL, 0.15 mol) and anhydrous methylene chloride (750 mL). Cool the solution to -75°C and add dimethylsulfoxide (21.3 mL, 0.30 mol) dropwise while maintaining the temperature below -55°C. Continue stirring for 5 minutes and then add the protected cytidine formed above in step a (54 g, 0.10 mol) in anhydrous methylene chloride (250 mL) over 10 minutes. Stir for 30 minutes at -75°C and add triethylamine (75.5 mL, 0.54 mol). Remove the ice bath allowing the reaction to warm to room temperature. After 1 hour at room temperature, dilute the reaction with an equal volume of diethyl ether and stir for an additional hour. Pour the mixture onto silica gel (500 mL) in a fritted funnel and elute with diethyl ether (1 L) followed by methylene chloride (1 L). Concentrate the diethyl ether wash and treat with 10% chloroform/hexane (300 mL). Filter the solid and dry to provide 31.6 g as a white powder. Concentrate the methylene chloride wash and recrystallize the residue from 10% chloroform/hexane (300 mL) to provide an additional 12.5 g for a total of 48.6 g of the title compound (90% yield). This compound readily hydrates at the C-2' position to form the ketone hydrate. It should be protected from prolonged exposure to moisture;
¹H NMR (CDCl₃) δ 0.99-1.16 (m, 28), 3.13 (s, 3), 3.14 (s, 3), 3.95-4.03 (m, 1), 4.06-4.22 (m, 2), 4.93 (s, 1), 5.22 (d, 1, J = 8.0 Hz), 6.02 (d, 1, J = 7.2 Hz), 7.29 (d, 1, J = 7.2 Hz), 8.82 (s, 1); MS (CI/CH₄) m/z 539 (MH⁺).
Anal. Calcd for C₂₄H₄₂N₄O₆Si₂.1/15 CHCl₃: C, 52.85; H, 7.75; N, 10.24.
Found: C, 52.72; H, 7.86; N, 10.24.

### Example 2

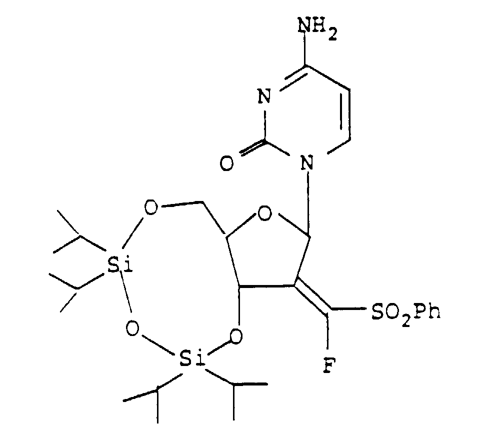

### (2'Z)-2'-deoxy-2'-[fluoro(phenylsulfonyl)methylene]-3',5'-O-[1,1,3,3-tetrakis(1-methylethyl)-1,3-disiloxanediyl] cytidine.

Scheme A, steps c and d; Under a nitrogen atmosphere, cool the fluoromethylphenyl sulfone (19.4 g, 0.11 mol) in anhydrous tetrahydrofuran (800 mL) to -70°C and add diethyl chlorophosphate (16 mL, 0.11 mol) via syringe. Next, slowly add 1M lithium bis(trimethylsilyl)amide (200 mL, 0.20 mol) using a dropping funnel. After complete addition maintain the reaction at -65°C for 1 hour. Add a solution of the above prepared ketone (40 g, 0.074 mol in 200 mL of tetrahydrofuran) using an addition funnel and maintain the temperature at -60°C. After complete addition, warm to 0°C for 30 minutes and then room temperature for 2.5 hours. Quench the reaction with saturated ammonium chloride (100 mL), dilute with diethyl ether (600 mL) and a small amount of water to dissolve the inorganic salts. Separate the layers and wash the organic phase with saturated sodium chloride. Combine the aqueous washes and back extract with diethyl ether (200 mL). Wash this organic phase with saturated sodium chloride. Combine the organic layers, dry over anhydrous magnesium sulfate, filter and concentrate under vacuum to provide a dark viscous oil (71.8 g). ¹⁹F NMR (CDCL3) shows four peaks, two from protected amino δ - 115.21 (Z isomer) and -119.70 (E isomer) and two peaks from the free amino derivative δ -115.62 (Z isomer) and - 119.40 (E isomer). The E/Z ratio is 10.4:1. Filter the crude sample through silica gel (1 L) with ethyl acetate (12 L). This step is optional before removing the amino protecting group. Concentrate the filtrate under vacuum to provide a viscous tan oil (46.8 g). Dissolve the oil in dioxane (200 mL) and add concentrated ammonium hydroxide (100 mL). Stir the reaction overnight. Then remove the solvent under vacuum and azeotrope the residue with ethanol (2 X 300 mL) to remove any residual water. Purify the product by flash chromatography (1.4 L silica gel, 5% hexane/ethyl acetate) to provide the E isomer (20 g). Purify isolated impure material (16 g) from flash chromatography by Prep HPLC (ethyl acetate) to provide additional E isomer (11.4 g) for a total of 31.4 g (66.3% yield) of the title compound. Recrystallize from hexane to provide a white powder, mp waxes at 135°C, clears at 145°C;
¹H NMR (CDCl₃) δ 0.97-1.11 (m, 28), 3.93-4.03 (m, 2), 4.09-4.17 (m, 1), 5.68 (d, 1, J = 7.2 Hz), 5.72 (br s, 2), 6.43 (t, 1, J = 2.0 Hz), 7.33 (d, 1, J = 7.5 Hz), 7.46-7.65 (m, 5); ¹⁹F NMR (CDCl₃) δ -119.22 (s); MS (CI/CH₄) m/z 640 (MH⁺).
Anal. Calcd for C₂₈H₄₂FN₃O₇SSi₂: C, 52.56; H, 6.61; N, 6.57.
Found: C, 52.40; H, 6.96; N, 6.36.

### Example 3

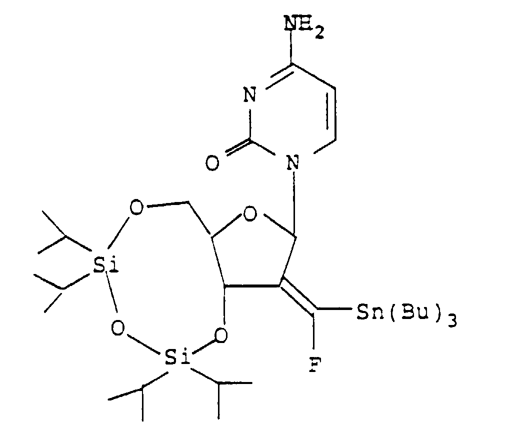

### (2'Z)-2'-deoxy-2'-[fluoro(tributylstannyl)methylene]-3',5'-O-[1,1,3,3-tetrakis(1-methylethyl)-1,3-disiloxanediyl] cytidine.

Scheme A, step e; Dissolve the above prepared fluorovinyl sulfone (26 g, 0.0406 mol) in benzene (300 mL) and reflex without a condenser for 15 minutes. Cool the reaction and add tributyltin hydride (32.6 mL, 0.122 mol) and azobisisobutyronitrile (500 mg). Reflux the reaction for 18 hours. Concentrate the reaction under vacuum, and purify the residue by flash chromatography (1.4 L silica gel, 4% methanol/methylene chloride, 4 L, followed by 6% methanol/methylene chloride) to provide the title compound (26.5 g, 82.8% yield) as a yellow foam; ¹H NMR (CDCl₃) δ 0.87 (t, 9), 0.94-1.17 (m, 34), 1.22-1.35 (m, 6), 1.38-1.50 (m, 6), 3.78-3.88 (m, 2), 3.96-4.04 (m, 1), 5.18 (br s, 1), 5.82 (d, 1, J = 7.5 Hz), 6.76 (br s, 1), 7.21 (d, 1, J = 7.7 Hz); ¹⁹F NMR (CDCl₃) δ -92.27 (s, 84%) and (d, 16%, J_{Sn-F} = 219 Hz); MS (CI/CH₄) m/z 790 (MH⁺).

### Example 4

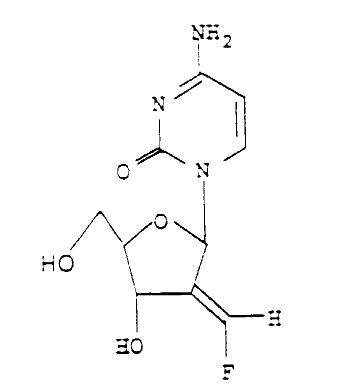

### (E)-2'-deoxy-2'(fluoromethylene)cytidine

Scheme A, step f; Dissolve the (fluorovinyl)stannane (26 g, 0.033 mol) formed above and potassium fluoride (9.6 g, 0.165 mol) in methanol (300 mL) and reflux for 24 hours. After cooling, partially concentrate the solution was under vacuum, add silica gel (75 mL) and then concentrate the mixture under vacuum to a free flowing powdery solid. Purify by filtering through silica gel (1 L) with 50% ethyl acetate/hexane (2 L), followed by 10% methanol/ethyl acetate (2 L) and 20% methanol/ethyl acetate (8 L) to provide 9.3 g of compound as a white solid [note- a lower R_{f} material visible by potassium permanganate stain, elutes with the later fractions. Trituration with diethyl ether lowers the concentration but traces still remain. Partition the product between water and diethyl ether and then lyophilize the aqueous layer to purify]. Recrystallize from methanol/ethyl acetate (120 mL) to yield 4.16 g and a second drop to yield 1.66 g of the title compound (6.26 g total, 68.7%) as white crystals, mp 166°C (foams); ¹H NMR (DMSO-d₆) δ 3.48-3.62 (m, 2), 3.73-3.78 (m, 1), 4.73-4.78 (m, 1), 4.95 (t, 1, J = 5.6 Hz), 5.65 (d, 1, J = 6.9 Hz), 5.73 (d, 1, J = 7.6 Hz), 6.65-6.68 (m, 1), 6.77 (dt, 1, J = 8.13, 2.0 Hz), 7.25 (br s, 1), 7.54 (d, 1, J = 7.3 Hz); ¹⁹F NMR (DMSO-d₆) δ -130.05 (d, J = 80.9 Hz); MS NEG (CI/CH₄) 257 (M⁻.).
Anal. Calcd for C₁₀H₁₂FN₃O₄: C, 46.70; H, 4.70; N, 16.34.
Found: C, 46.81; H, 4.71; N, 16.18.

### Example 5

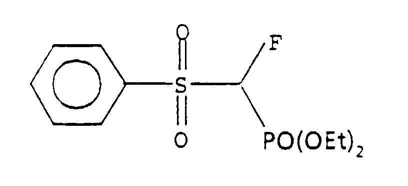

### Diethyl 1-fluoro-1-(phenylsulfonyl)methanephosphonate

Scheme B, step a; Charge a 3 neck 100 mL round bottom flask flushed with nitrogen with diethyl 1-chloro-1-(phenylsulfide)methanephosphonate ( 62 mmol), cesium fluoride ( 126 mmol) and a mixture of poly(ethylene glycol)-200 and acetonitrile (38 mL of in a 1:2 ratio). Heat the reaction to 80°C with stirring for 2 hours. Cool the reaction, dilute with water (125 mL) and extract with chloroform (2 X 125 mL). Combine the organic extracts, wash with water (50 mL), dry over anhydrous magnesium sulfate, filter and concentrate under vacuum to yield diethyl 1-fluoro-1-(phenylsulfide)methanephosphonate.

Scheme B, step b; Dissolve the crude diethyl 1-fluoro-1-(phenylsulfide)methanephosphonate in methanol (85 mL) and cool to 0°C. Add a solution of potassium peroxymonosulfate (63 mmol in 85 mL water) slowly with stirring. The temperature increases to approximately 55°C. After cooling, stir the reaction for 4 hours and then concentrate the reaction under vacuum. Suction filter the remaining slurry through diatomaceous earth and rinse with chloroform. Separate the layers and extract the aqueous with chloroform. Combine the organic extracts, dry over anhydrous magnesium sulfate, filter and concentrate under vacuum. The residue is then purified by techniques will known to one skilled in the art, such as flash chromatography to provide the title compound.

### Example 6

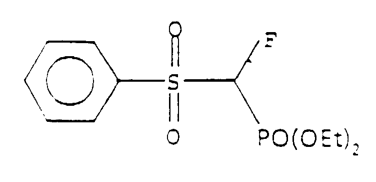

### Diethyl 1-fluoro-1-(phenylsulfonyl)methanephosphonate

Scheme C, step a; Cool a solution of diethyl 1-(phenylsulfide)methanephosphonate (20 g, 76.8mmol) and triethylamine trihydrofluoride (37 g, 230 mmol) in tetrahydrofuran (200 mL) to -78°C. Electrolysis is performed at platinum electrodes (3.8 X 12 cm) for 15 minutes at 0.5 A and then increased to 1.0 A for 6.25 hours and then stopped. After sitting overnight, continue the electrolysis for an additional 3 hours at 1.0 Afor a total time of 9.5 hours. Dilute the reaction with diethyl ether (200 mL) and rinse with 2 molar ammonium hydroxide. Wash the aqueous and extract with diethyl ether (200 mL). Combine the organic phases and dry over anhydrous magnesium sulfate. Filter and concentrate to provide the crude material as a brown oil (27.4 g). Purify the crude material by passing through silica gel (500 g, 60-200 mesh) with ethyl acetate:hexane (1:6, 4 L then 1:3, 2 L) followed by ethyl acetate to provide the diethyl 1-fluoro-1-(phenylsufide)methanephosphonate (10.7 g, 50%).

Scheme C, step b; Oxidize the diethyl 1-fluoro-1-(phenylsufide)methanephosphonate (8.4 g, 86% pure) in a manner analogous to that in example 5, step b by dissolving in methanol (200 mL) and treating with potassium peroxymonosulfate (35g in 300 mL water) to provide the title compound.

### Example 7

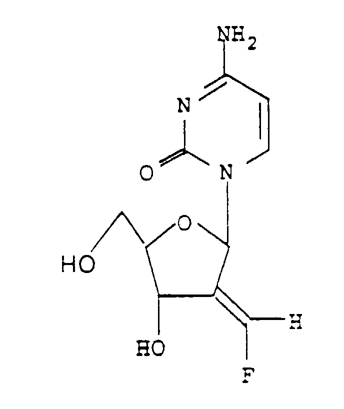

### (E)-2'-deoxy-2'(fluoromethylene)cytidine

Scheme A, step f [sequential method]; Dissolve (2'Z)-2'-deoxy-2'-[fluoro(tributylstannyl)methylene]-3',5'-O-[1,1,3,3-tetrakis(1-methylethyl)-1,3-disiloxanediyl]cytidine (8 g, 0.01 mol) prepared in example 3 in methylene chloride (200 mL), add activated silica gel (approximately 50 g, 60-200 mesh) and stir until TLC indicates the protolysis is complete. Filter the reaction and concentrate the filtrate under vacuum to yield the protected exocyclic vinylfluoride. Dissolve the protected exocyclic vinylfluoride ( 0.01 mol) in tetrahydrofuran (200 mL) and treat with tetrabutylammonium fluoride ( 0.025 mol). Stir the reaction until TLC indicates removal of the 3', 5' protecting group is complete. The product is then isolated and purified by techniques well known to one of ordinary skill in the art to provide the title compound.

## Claims

1. A process for preparing a compound of the formula 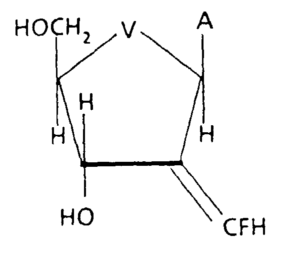 wherein
V is oxy or methylene and
A is a radical of the formula 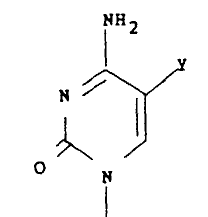
wherein Y is hydrogen, C₁-C₄ alkyl or C₁-C₄ alkoxy comprising the steps of:
(a) reacting a compound of the formula 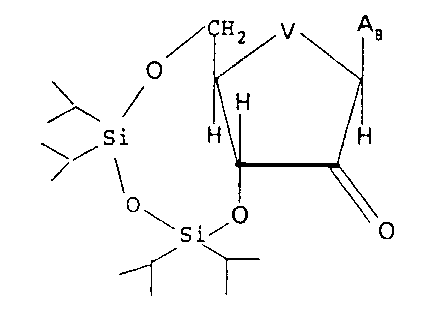 wherein A_{B} is a radical of the formula 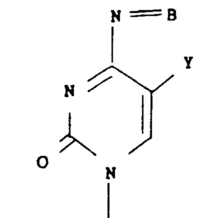 and B is a nitrogen blocking group of the formula CR₁NR₂R₃ wherein R₁ is a hydrogen or C₁-C₄ alkyl and R₂ and R₃ are independently C₁-C₄ alkyl, with a phosphonate ylid of the formula (X)₂OP=CF(SO₂Ar) wherein Ar is an aryl group and X is a phenoxy or C₁-C₄ alkoxy to produce an exocyclic fluorovinyl sulfone;
(b) reacting the exocyclic fluorovinyl sulfone with a base or a weak acid to produce a deprotected amine;
(c) reacting the deprotected amine with a stannylating reagent of the formula (R)₃SnH wherein R is aryl or C₁-C₄ alkyl to produce an exocyclic (fluorovinyl)stannane;
(d) reacting the exocyclic (fluorovinyl)stannane with a protolysis agent and, either concomitantly or sequentially, reacting the silyl protecting group with an acid or a fluoride ion source.

2. A process for preparing a compound of the formula 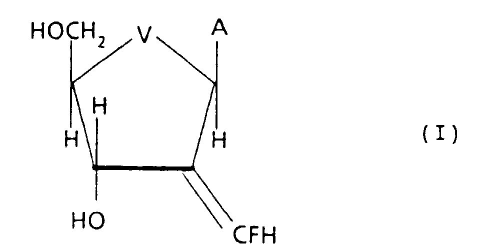 wherein
V is oxy or methylene and
A is a radical of the formula 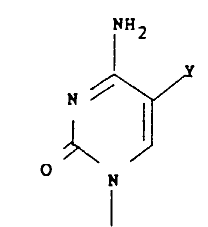
wherein Y is hydrogen, C₁-C₄ alkyl or C₁-C₄ alkoxy comprising the steps of:
(a) reacting a compound of the formula 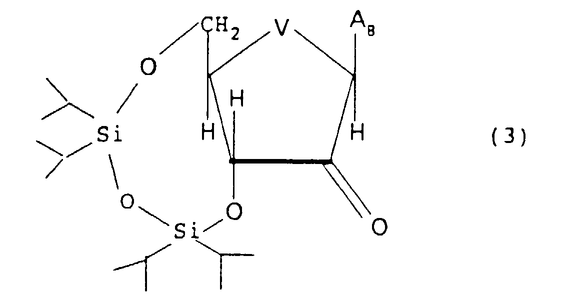 wherein A_{B} is a radical of the formula 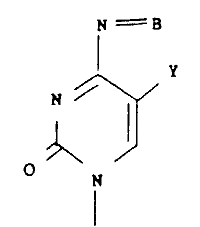 and B is a nitrogen blocking group of the formula CR₁NR₂R₃ wherein R₁ is a hydrogen or C₁-C₄ alkyl and R₂ and R₃ are independently C₁-C₄ alkyl, with a base or weak acid to produce a deprotected amine;
(b) reacting the deprotected amine with a phosphonate ylid of the formula (X)₂OP=CF(SO₂Ar) wherein Ar is an aryl group and X is a phenoxy or C₁-C₄ alkoxy to produce an exocyclic fluorovinyl sulfone;
(c) reacting the exocyclic fluorovinyl sulfone with a stannylating reagent of the formula (R)₃SnH wherein R is aryl or C₁-C₄ alkyl to produce an exocyclic (fluorovinyl)stannane;
(d) reacting the exocyclic (fluorovinyl)stannane with a protolysis agent and, either concomitantly or sequentially, reacting the silyl protecting group with a acid or a fluoride ion source.

3. A compound of the formula 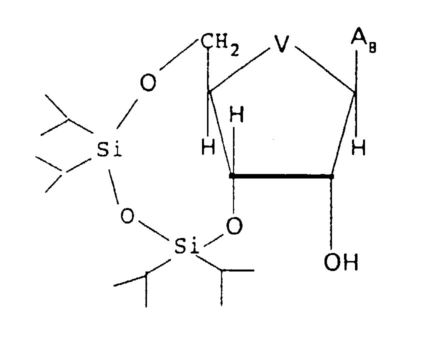 wherein
V is oxy or methylene and
A_{B} is a radical of the formula 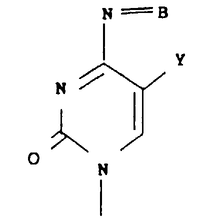
wherein Y is hydrogen, C₁-C₄ alkyl or C₁-C₄ alkoxy and B is a nitrogen blocking group of the formula CR₁NR₂R₃ wherein R₁ is a hydrogen or C₁-C₄ alkyl and R₂ and R₃ are independently C₁-C₄ alkyl.

4. A compound of the formula 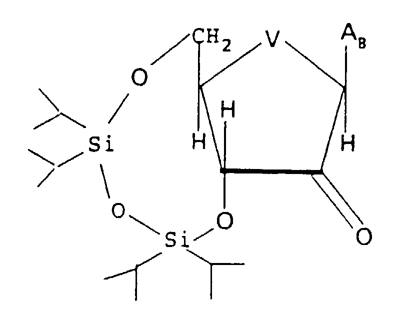 wherein
V is oxy or methylene and
A_{B} is a radical of the formula 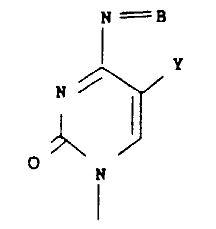
wherein Y is hydrogen, C₁-C₄ alkyl or C₁-C₄ alkoxy and B is a nitrogen blocking group of the formula CR₁NR₂R₃ wherein R₁ is a hydrogen or C₁-C₄ alkyl and R₂ and R₃ are independently C₁-C₄ alkyl.

5. A compound of the formula 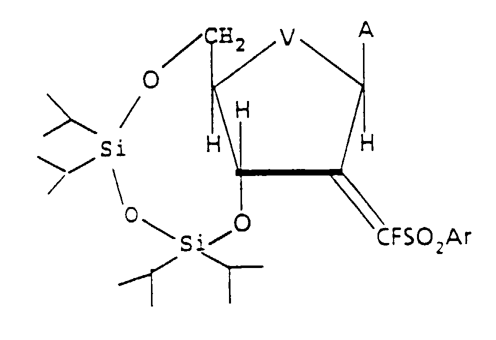 wherein
V is oxy or methylene,
Ar is an aryl group and
A is a radical of the formula 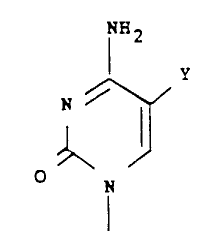
wherein Y is hydrogen, C₁-C₄ alkyl or C₁-C₄ alkoxy.

6. A compound of the formula 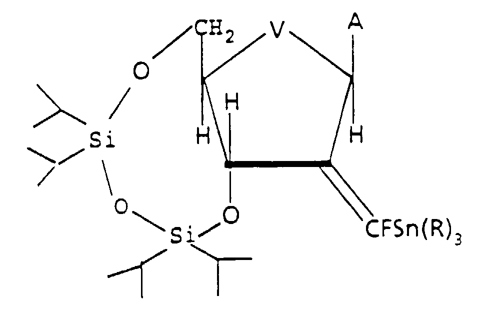 wherein
V is oxy or methylene,
R is C₁-C₄ alkyl or aryl and
A is a radical of the formula 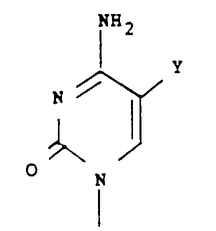
wherein Y is hydrogen, C₁-C₄ alkyl or C₁-C₄ alkoxy.

## Patentansprüche

1. Verfahren zur Herstellung einer Verbindung der Formel, 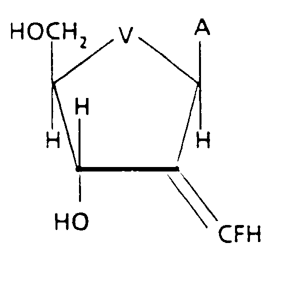 in der
V ein Sauerstoffatom oder eine Methylengruppe darstellt und
A einen Rest der Formel 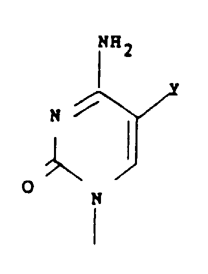
bedeutet, wobei Y ein Wasserstoffatom, einen C₁-C₄-Alkyl- oder C₁-C₄-Alkoxyrest darstellt, umfassend die Stufen:
(a) Umsetzen einer Verbindung der Formel 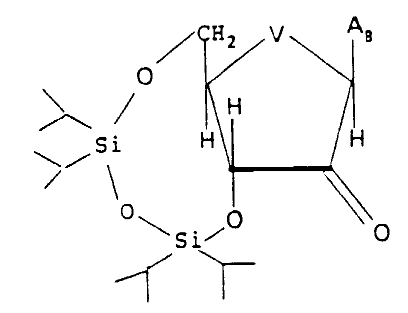 in der A_{B} einen Rest der Formel 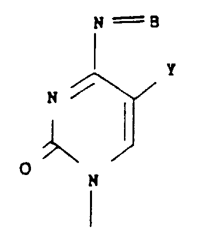 bedeutet und B eine Stickstoffschutzgruppe der Formel CR₁NR₂R₃ bedeutet, wobei R₁ ein Wasserstoffatom oder einen C₁-C₄-Alkylrest und R₂ und R₃ unabhängig voneinander einen C₁-C₄-Alkylrest darstellen,
mit einem Phosphonatylid der Formel (X)₂OP=CF(SO₂Ar), in der Ar einen Arylrest und X eine Phenoxygruppe oder einen C₁-C₄-Alkoxyrest darstellen, wobei ein exocyclisches Fluorvinylsulfon hergestellt wird;
(b) Umsetzen des exocyclischen Fluorvinylsulfons mit einer Base oder einer schwachen Säure, wobei ein ungeschütztes Amin erhalten wird;
(c) Umsetzen des ungeschützten Amins mit einem Stannylierungsmittel der Formel (R)₃SnH, in der R einen Aryl- oder einen C₁-C₄-Alkylrest darstellt, wobei ein exocyclisches (Fluorvinyl)stannan hergestellt wird;
(d) Umsetzen des exocyclischen (Fluorvinyl)stannans mit einem Protolysemittel und, entweder gleichzeitig oder nacheinander, Umsetzen der Silylschutzgruppe mit einer Säure oder einer Fluoridionenquelle.

2. Verfahren zur Herstellung einer Verbindung der Formel, 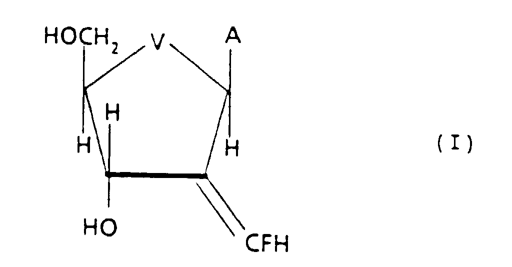 in der
V ein Sauerstoffatom oder eine Methylengruppe darstellt und
A einen Rest der Formel 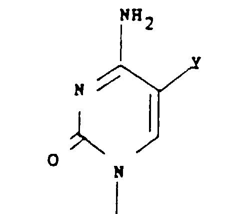
bedeutet, wobei Y ein Wasserstoffatom, einen C₁-C₄-Alkyl- oder C₁-C₄-Alkoxyrest darstellt, umfassend die Stufen:
(a) Umsetzen einer Verbindung der Formel 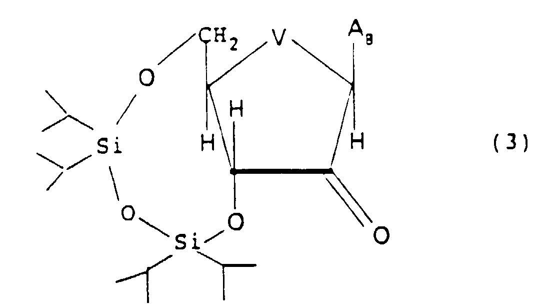 in der A_{B} einen Rest der Formel 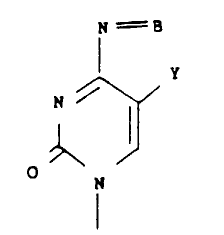 bedeutet und B eine Stickstoffschutzgruppe der Formel CR₁NR₂R₃ bedeutet, wobei R₁ ein Wasserstoffatom oder einen C₁-C₄-Alkylrest und R₂ und R₃ unabhängig voneinander einen C₁-C₄-Alkylrest darstellen,
mit einer Base oder einer schwachen Säure, wobei ein ungeschütztes Amin hergestellt wird:
(b) Umsetzen des ungeschützten Amins mit einem Phosphonatylid der Formel (X)₂OP=CF(SO₂Ar), in der Ar einen Arylrest und X eine Phenoxygruppe oder einen C₁-C₄-Alkoxyrest darstellen, wobei ein exocyclisches Fluorvinylsulfon hergestellt wird; (c) Umsetzen des exocyclischen Fluorvinylsulfons mit einem Stannylierungsmiitel der Formel (R)₃SnH, in der R einen Aryl- oder einen C₁-C₄-Alkylrest darstellt, wobei ein exocyclisches (Fluorvinyl)stannan hergestellt wird;
(d) Umsetzen des exocyclischen (Fluorvinyl)stannans mit einem Protolysemittel und, entweder gleichzeitig oder nacheinander, Umsetzen der Silylschutzgruppe mit einer Säure oder einer Fluoridionenquelle.

3. Verbindung der Formel 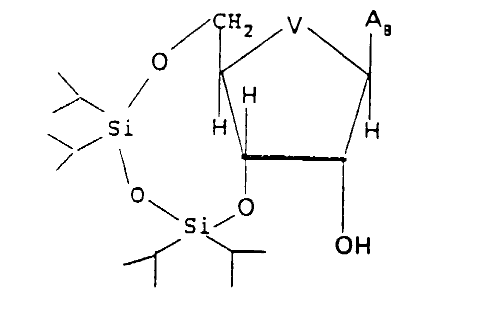 in der
V ein Sauerstoffatom oder eine Methylengruppe darstellt und
A_{B} einen Rest der Formel 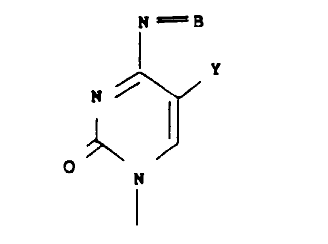
bedeutet, wobei Y ein Wasserstoffatom, einen C₁-C₄-Alkyl- oder C₁-C₄-Alkoxyrest darstellt und B eine Stickstoffschutzgruppe der Formel CR₁NR₂R₃ bedeutet, in der R₁ ein Wasserstoffatom oder einen C₁-C₄-Alkylrest und R₂ und R₃ unabhängig voneinander einen C₁-C₄-Alkylrest darstellen.

4. Verbindung der Formel 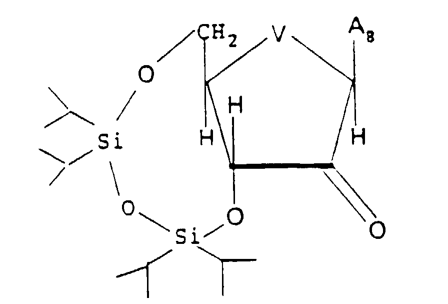 in der
V ein Sauerstoffatom oder eine Methylengruppe darstellt und
A_{B} einen Rest der Formel 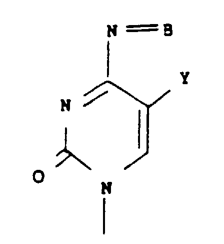
bedeutet, wobei Y ein Wasserstoffatom, einen C₁-C₄-Alkyl- oder C₁-C₄-Alkoxyrest darstellt und B eine Stickstoffschutzgruppe der Formel CR₁NR₂R₃ bedeutet, in der R₁ ein Wasserstoffatom oder einen C₁-C₄-Alkylrest und R₂ und R₃ unabhängig voneinander einen C₁-C₄-Alkylrest darstellen.

5. Verbindung der Formel 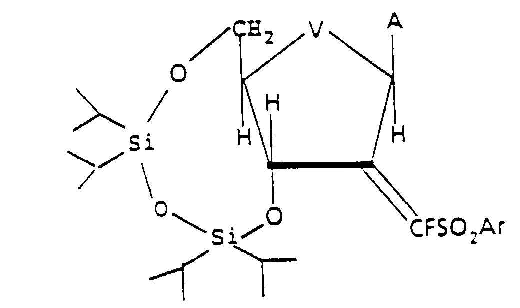 in der
V ein Sauerstoffatom oder eine Methylengruppe darstellt
Ar einen Arylrest darstellt und
A einen Rest der Formel 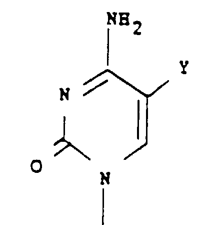
bedeutet, wobei Y ein Wasserstoffatom, einen C₁-C₄-Alkyl- oder C₁-C₄-Alkoxyrest darstellt.

6. Verbindung der Formel 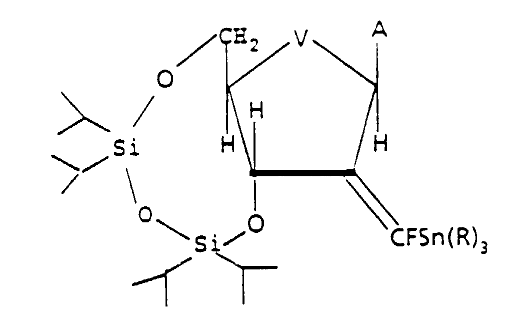 in der
V ein Sauerstoffatom oder eine Methylengruppe darstellt
R einen C₁-C₄-Alkyl- oder einen Arylrest darstellt und
A einen Rest der Formel 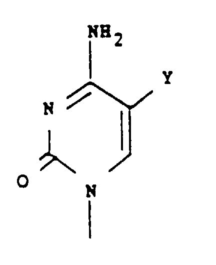
bedeutet, wobei Y ein Wasserstoffatom, einen C₁-C₄-Alkyl- oder C₁-C₄-Alkoxyrest darstellt.

## Revendications

1. Procédé de préparation d'un composé de formule 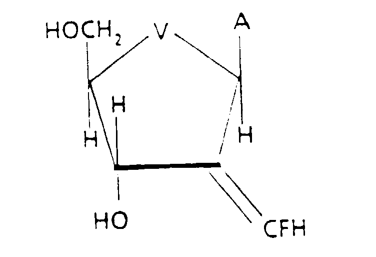 dans laquelle
V représente un groupe oxy ou un groupe méthylène et
A représente un radical de formule 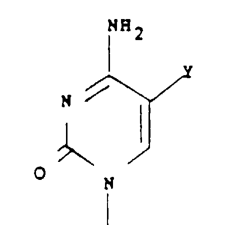
dans laquelle Y représente un atome d'hydrogène, un groupe alkyle en C₁₋₄ ou un groupe alcoxy en C₁₋₄, comprenant les étapes consistant à :
(a) faire réagir un composé de formule 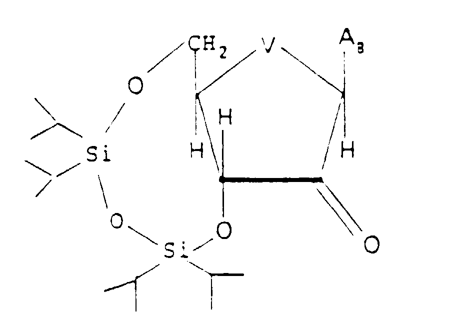 dans laquelle A_{B} représente un radical de formule 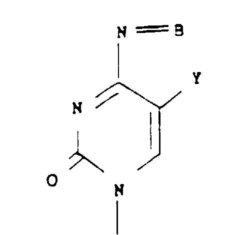 et B représente un groupe bloquant l'atome d'azote de formule CR₁NR₂R₃, dans laquelle R₁ représente un atome d'hydrogène ou un groupe alkyle en C₁₋₄ et R₂ et R₃ représentent chacun indépendamment un groupe alkyle en C₁₋₄, avec un ylure de phosphonate de formule (X)₂OP=CF(SO₂Ar) dans laquelle Ar représente un groupe aryle et X représente un groupe phénoxy ou alcoxy en C₁₋₄ pour obtenir une fluorovinylsulfone exocyclique ;
(b) faire réagir une fluorovinylsulfone exocyclique avec une base ou un acide faible pour produire une amine déprotégée ;
(c) faire réagir l'amine déprotégée avec un réactif de stannylation de formule (R)₃SnH, dans laquelle R représente un groupe aryle ou un groupe alkyle en C₁₋₄ pour produire un (fluorovinyl)stannane exocyclique;
(d) faire réagir le (fluorovinyl)stannane exocyclique avec un agent de protolyse et, en même temps ou l'un après l'autre, faire réagir le groupe protecteur silyle avec un acide ou une source d'ions fluorure.

2. Procédé de préparation d'un composé de formule 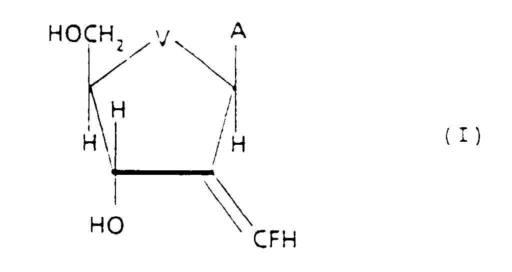 dans laquelle
V représente un groupe oxy ou méthylène et
A représente un radical de formule 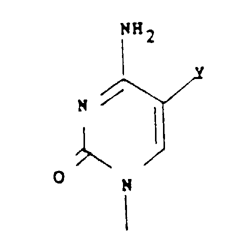
dans laquelle Y représente un atome d'hydrogène, un groupe alkyle en C₁₋₄ ou alcoxy en C₁₋₄, comprenant les étapes consistant à :
(a) faire réagir un composé de formule 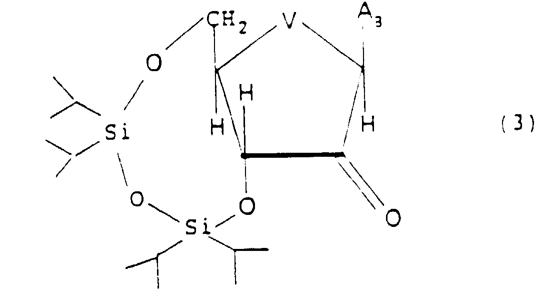 dans laquelle A_{B} représente un radical de formule 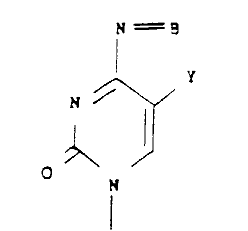 et B représente un groupe bloquant l'atome d'azote de formule CR₁NR₂R₃, dans laquelle R₁ représente un atome d'hydrogène ou un groupe alkyle en C₁₋₄ et R₂ et R₃ représentent chacun indépendamment un groupe alkyle en C₁₋₄, avec une base ou un acide faible pour obtenir une amine déprotégée ;
(b) faire réagir l'amine déprotégée avec un ylure de phosphonate de formule (X)₂OP=CF(SO₂Ar), dans laquelle Ar représente un groupe aryle et X représente un groupe phénoxy ou alcoxy en C₁₋₄ pour produire une fluorovinylsulfone exocyclique ;
(c) faire réagir la fluorovinylsulfone exocyclique avec un réactif de stannylation de formule (R)₃SnH, dans laquelle R représente un groupe aryle ou un groupe alkyle en C₁₋₄ pour produire un (fluorovinyl)stannane exocyclique ;
(d) faire réagir le (fluorovinyl)stannane exocyclique avec un agent de protolyse et, en même temps ou successivement, faire réagir le groupe protecteur silyle avec un acide ou une source d'ions fluorure.

3. Composé de formule 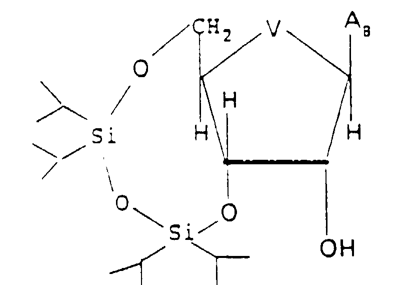 dans laquelle
V représente un groupe oxy ou méthylène et
A_{B} représente un radical de formule 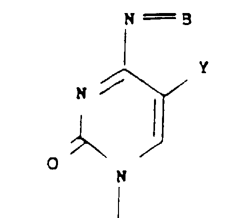
dans laquelle Y représente un atome d'hydrogène, un groupe alkyle en C₁₋₄ ou alcoxy en C₁₋₄ et B représente un groupe bloquant l'atome d'azote de formule CR₁NR₂R₃, dans laquelle R₁ représente un atome d'hydrogène ou un groupe alkyle en C₁₋₄ et R₂ et R₃ représentent chacun indépendamment un groupe alkyle en C₁₋₄.

4. Composé de formule 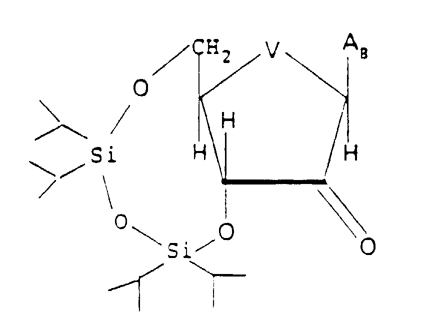 dans laquelle
V représente un groupe oxy ou méthylène et
A_{B} représente un radical de formule 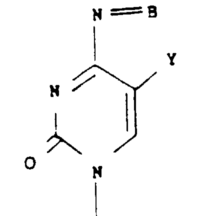
dans laquelle Y représente un atome d'hydrogène, un groupe alkyle en C₁₋₄ ou alcoxy en C₁₋₄ et B représente un groupe bloquant l'atome d'azote de formule CR₁NR₂R₃, dans laquelle R₁ représente un atome d'hydrogène ou un groupe alkyle en C₁₋₄ et R₂ et R₃ représentent chacun indépendamment un groupe alkyle en C₁₋₄.

5. Composé de formule 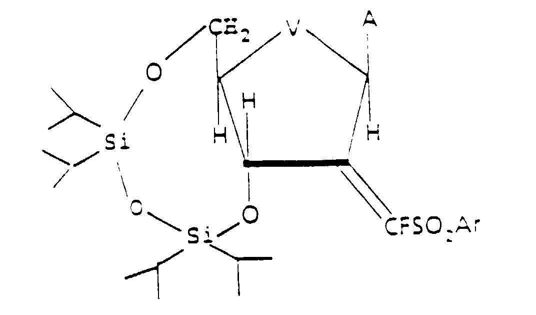 dans laquelle
V représente un groupe oxy ou méthylène,
Ar représente un groupe aryle et
A représente un radical de formule 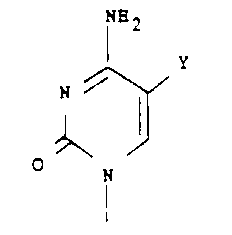
dans laquelle Y représente un atome d'hydrogène, un groupe alkyle en C₁₋₄ ou un groupe alcoxy en C₁₋₄.

6. Composé de formule 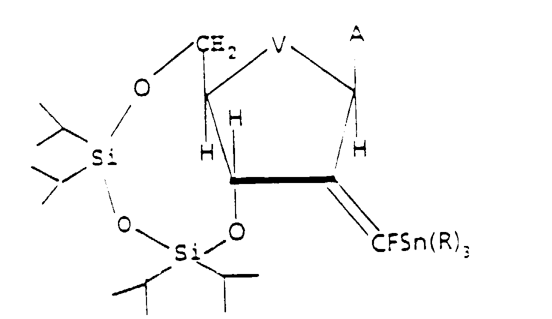 dans laquelle
V représente un groupe oxy ou méthylène,
R représente un groupe alkyle ou aryle en C₁₋₄ et
A représente un radical de formule 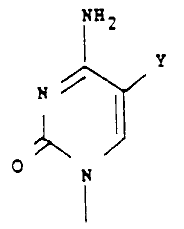
dans laquelle Y représente un atome d'hydrogène, un groupe alkyle en C₁₋₄ ou un groupe alcoxy en C₁₋₄.
